# EUROPÄISCHE PATENTSCHRIFT

(11) **EP 1 777 266 B1**
(45) Veröffentlichungstag und Bekanntmachung des Hinweises auf die Patenterteilung: **26.10.2011**
(21) Anmeldenummer: 06014211.4
(22) Anmeldetag: 08.07.2006
(51) Int. Cl.: C09B 41/00, C09B 45/14, C09B 45/22, C09B 67/20, C09B 67/22, C09D 11/02, C09D 17/00, G02B 5/22, G03F 7/00

(54) **Verfahren zur Herstellung von Metall-Verbindungen einer Azo-Verbindung in Gegenwart von Impfkristallen**
Process for the preparation of metal compounds of azo compounds in the presence of seeding crystals
Procédé pour la préparation de composés metallisés azoiques en présence de cristaux de germination

(30) Priorität: 19.07.2005 DE 102005033580
(43) Veröffentlichungstag der Anmeldung: 25.04.2007
(73) Patentinhaber: LANXESS Deutschland GmbH, 51369 Leverkusen (DE)
(72) Erfinder: Feldhues, Ulrich, Dr., 51465 Bergisch Gladbach (DE); Linke, Frank, 51069 Köln (DE); Göbel, Ronald, 51371 Leverkusen (DE); Endert, Sabine, 42327 Wuppertal (DE); Pfützenreuter, Dirk, 51399 Burscheid (DE)

(56) Entgegenhaltungen:
- EP-A- 0 994 162
- EP-A- 1 146 087
- EP-A- 1 174 473
- DE-A1- 4 432 546
- DE-A1- 10 328 999
- US-A- 3 944 540

## Beschreibung

Die Erfindung betrifft ein Verfahren zur Herstellung von Metall-Verbindungen einer Azo-Verbindung in Gegenwart von Impfkristallen, die Verwendung der Metallverbindungen als Pigmente und die Verwendung der Pigmente.

Metallkomplexpigmente von Metallen mit einer Azo-Verbindung der folgenden Formel 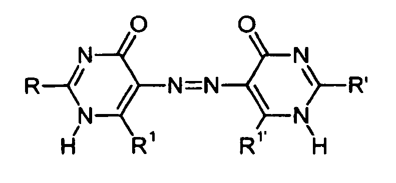 in der
R und R' unabhängig voneinander für OH, NH₂, NH-CN, Arylamino oder Acylamino stehen und
R¹ und Ru^{1'} unabhängig voneinander -OH oder -NH₂ bedeuten,
sowie Einlagerungsverbindungen daraus sind in der Literatur umfangreich beschrieben, z.B.:
- DE-A-2 064 093
- US-A-4,622,391
- EP 0 994 162 A1
- EP 0 994 163 A1
- EP 0 994 164 A1
- DE 103 28 999 A1.

Es ist bekannt, dass es bei der Herstellung der oben beschriebenen Metallverbindungen bzw. Einlagerungsverbindungen daraus zu relativ starken Schwankungen der Produkteigenschaften kommt. Insbesondere unter Produktionsbedingungen im industriellen Maßstab insbesondere im Batch-Verfahren unterliegen bestimmte Parameter des erhaltenen Materials, wie z.B. die spezifische Oberfläche nach BET, mehr oder weniger starken Schwankungen. Dies ist natürlich nachteilig, da die Abnehmer der genannten Verbindungen eine konstante Produktqualität wünschen. Weiterhin werden insbesondere für die Herstellung von Farbfiltern für Flüssigkristallanzeigen Produktqualitäten mit hohen BET-Oberflächen gewünscht.

Die geschilderten Nachteile des Batch-Verfahrens werden ausführlich in der EP-A-1142960 beschrieben. Das dort beschriebene kontinuierliche Herstellungsverfahren erfordert einen erheblichen zusätzlichen apparativen Aufwand, insbesondere mehrere Eduktbehälter, einen Mischreaktor und Mengen- und pH-gesteuerte Regel-, Pump- und SicherheitsSysteme, die extrem genau aufeinander abgestimmt sein müssen.

Eine gewisse Vereinheitlichung der Produktqualität (Reproduzierbarkeit) ist möglich, durch einen Temperschritt, wie er beispielsweise in der EP-A1-0994162 oder der DE 10328999 A 1 beschrieben ist. Dabei nimmt man jedoch eine Verringerung der spezifischen Oberfläche nach BET in Kauf, was insbesondere für die Anwendung zur Herstellung von Farbfiltern für Flüssigkristallanzeigen nachteilig sein kann.

Aufgabe der Erfindung war es somit, ein Verfahren zur Herstellung der erwähnten Metallverbindungen zu finden, dass die Reproduzierbarkeit der Herstellung der Metallverbindungen oder Einlagerungsverbindungen daraus erhöht und insbesondere die reproduzierbare Bildung eines Produktes ermöglicht, das über eine hohe BET-Oberfläche verfügt. Schwankungen in der Produktqualität sollten möglichst klein sein.

Die Erfindung betrifft ein Verfahren zur Herstellung von Nickel-Verbindungen einer Azo-Verbindung die in Form ihrer freien Säure der Formel: 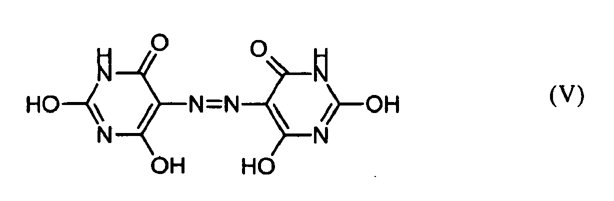 oder dazu tautomeren Strukturen
entspricht,
und die Melamin eingelagert enthalten,
mit einer spezifischen Oberfläche nach BET von mindestens 180 m²/g,
**dadurch gekennzeichnet, dass** die Herstellung in Gegenwart von Impfkristallen erfolgt.

Erfindungsgemäß werden unter Metall-Verbindungen einer Azo-Verbindung der Formel (V) insbesondere Metallkomplexverbindungen der Azo-Verbindung der Formel (V) und/oder salzartige Metallverbindungen der Azo-Verbindung der Formel (V) verstanden. Die Azo-Verbindung der Formel (V) liegt in den erfindungsgemäß hergestellten Metall-Verbindungen in der Regel ein- oder mehrfach deprotoniert als Anion vor, wohingegen die Metalle als Kationen vorliegen, die salzartig bis komplexartig bzw. koordinativ (also mit kovalenten Bindungsanteilen) mit dem Anion der Azo-Verbindung der Formel (V) verbunden sind. Die Formel (V) zeigt die Azo-Verbindung in der nicht deprotonierten Form, d.h. in der freien Säureform. Die Herstellung dieser komplexartigen und/oder salzartigen Metallverbindungen beruht vorzugsweise auf der Umsetzung der sauer reagierenden Azoverbindungen der Formel (V) mit Metallverbindungen, wahlweise in Gegenwart von Basen, unter Bildung der Metall-Verbindungen einer Azo-Verbindung der Formel (V).

Die erfindungsgemäß hergestellten Metallverbindungen oder die Einlagerungsverbindungen daraus können auch als Hydrate vorliegen.

L₁ Wasserstoff, -OR₆, -SR₆, -NR₆R₇, -COOR₆, -CON%R₇, -CN, Alkyl, Cycloalkyl, Aryl, Aralkyl ist und
M₁ -OR₆, -SR₆, -NR₆R₇, -COOR₆, -CONR₆R₇, -CN, -SO₂R₈, 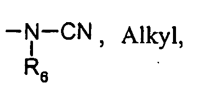 Cycloalkyl, Aryl oder Aralkyl bezeichnen,

oder die Substituenten M₁ und R₁ oder M₁ und R₂ einen 5- oder 6-gliedrigen Ring ausbilden können, und
R₁, R₂, R₅, R₆, R₇ und R₈ die oben angegebenen Bedeutungen besitzen.

Besonders bevorzugte erfindungsgemäß hergestellte Metallverbindungen sind dabei solche von Azo-Verbindungen, die in Form ihrer freien Säuren Strukturen der Formeln (II) oder (III) entsprechen 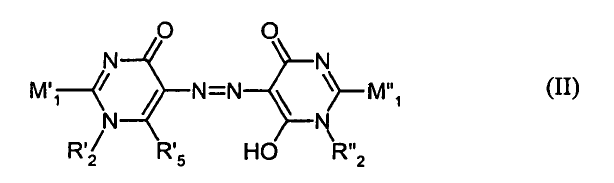 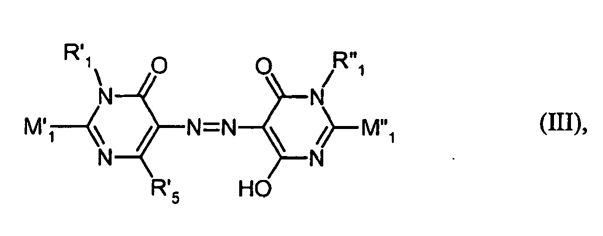 oder dazu tautomeren Formen,
in denen
R'₅ -OH oder -NH₂ bezeichnet,
R'₁, R"₁, R'₂ und R"₂ jeweils für Wasserstoff stehen und
M'₁ und M"₁ unabhängig voneinander für Wasserstoff, -OH, -NH₂, -NHCN, Arylamino oder Acylamino stehen.
Ganz besonders bevorzugte Metallverbindungen sind dabei solche von Azo-Verbindungen der Formel (I), die in Form ihrer freien Säure einer Struktur der Formel (IV) entsprechen 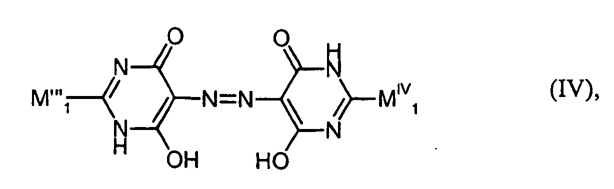 oder tautomeren Strukturen davon,
in denen
M"'₁ und M^{IV}₁ unabhängig voneinander OH und/oder NHCN bedeuten.

Besonders bevorzugt sind Metallverbindungen von Azo-Verbindungen der Formel: 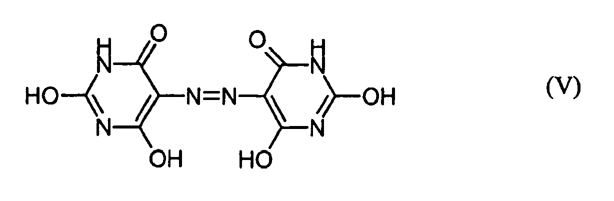 oder dazu tautomeren Strukturen.

In den vorstehenden Formeln haben die Substituenten vorzugsweise die folgenden Bedeutungen:
Substituenten in der Bedeutung von Alkyl bezeichnen vorzugsweise C₁-C₆-Alkyl, das beispielsweise durch Halogen, wie Chlor, Brom, Fluor, -OH, -CN, -NH₂ oder C₁C₆-Alkoxy substituiert sein kann. Darin bedeutet C₁-C₆₋Alkyl geradkettiges oder verzweigtes Alkyl mit 1 bis 6 Kohlenstoffatomen, wie Methyl, Ethyl, Propyl, Butyl, Pentyl, Hexyl einschließlich sämtlicher isomerer Formen davon.
Substituenten in der Bedeutung von Cycloalkyl bezeichnen vorzugsweise C₃-C₇-Cycloalkyl, insbesondere C₅-C₆-Cycloalkyl, das beispielsweise durch C₁-C₆-Alkyl, C₁-C₆-Alkoxy, Halogen wie Cl, Br, F, C₁-C₆-Alkoxy, -OH, -CN und NH₂ substituiert sein kann.
Als Metallverbindungen, worunter wie bereits dargelegt, salz- oder komplexartige Metallverbindungen verstanden werden, der Azoverbindungen der Formel (V) kommen vorzugsweise die Salze und Komplexe der Mono-, Di-, Tri- und Tetraanionen der Azoverbindungen der Formel (V) in Betracht.

Bevorzugt handelt es sich bei der Metallverbindung um den Azobarbitursäure-Nickel-1:1-Komplexes der Struktur 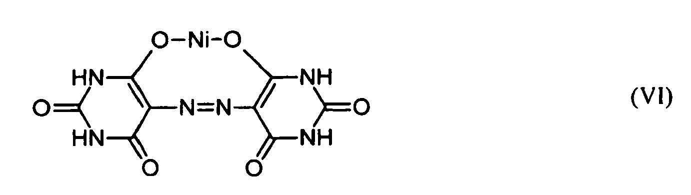 oder eine tautomere Struktur dazu.

Bei den erfindungsgemäß hergestellten Zusammensetzungen aus Metallverbindung und eingelagerter organischer Verbindung kann es sich um Einlagerungsverbindungen, Interkalationsverbindungen oder feste Lösungen handeln.

Bevorzugt handelt es sich um Einschlußverbindungen, Interkalationsverbindungen bzw. feste Lösungen eines Azobarbitursäure-Nickel-1:1-Komplexes der Struktur 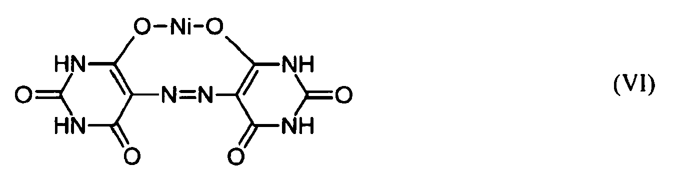 oder einer tautomeren Struktur dazu und mindestens einer anderen darin eingeschlossenen organischen Verbindung.

Besonders bevorzugt handelt es sich um Interkalationsverbindungen der vorstehend beschriebenen Metallverbindung der Formel (VI) mit Melamin in einem molaren Verhältnis von 1 : 2.

Im allgemeinen bilden die erfindungsgemäß hergestellten Metallverbindungen schichtförmigen Kristallgitter, bei dem die Bindung innerhalb einer Schicht im wesentlichen über Wasserstoffbrücken und/oder Metallionen erfolgt. Vorzugsweise handelt es sich dabei um Metallkomplexe, die ein Kristallgitter ausbilden, das aus im wesentlichen ebenen Schichten besteht.

Bevorzugt erfolgt die erfindungsgemäße Herstellung der Metallkomplexe der Azoverbindung der Formel (V) bzw. der Einlagerungsverbindungen davon in Gegenwart von Impfkristallen, die die gleiche chemische Struktur besitzen wie die nach dem erfindungsgemäßen Verfahren herzustellenden Metallverbindungen der Azoverbindung der Formel (V) bzw. die Einlagerungsverbindungen davon. Insbesondere werden, wenn das herzustellende Produkt eine Zusammensetzung aus einer Metallverbindung der Azoverbindung der Formel (V) und einer darin eingelagerten Verbindung ist, auch Impfkristalle einer solchen Einschlusszusammensetzung verwendet. Es hat sich dabei überraschend herausgestellt, dass die physikalischen Eigenschaften der verwendeten Impfkristalle nicht notwendigerweise die physikalischen Eigenschaften der herzustellenden Metallverbindungen bestimmen. So werden beispielsweise Metallverbindungen mit hoher spezifischer Oberfläche nach BET auch dann erhalten, wenn die eingesetzten Impfkristalle eine vergleichweise geringe spezifische Oberfläche nach BET aufweisen.

Bevorzugt erfolgt die erfindungsgemäße Herstellung in Gegenwart von 1 ppm - 10.000 ppm an Impfkristallen bezogen auf die theoretisch erhältliche Menge der herzustellenden Metallverbindung eines gegebenen Reaktionsansatzes, insbesondere von 10 ppm - 5.000 ppm, ganz besonders bevorzugt von 50 ppm - 3.000 ppm, insbesondere von 100 ppm - 2.000 ppm.

Erfindungsgemäß können vorteilhaft Metallverbindungen der Azoverbindungen der Formel (V), die vorzugsweise eine Verbindung eingelagert enthalten, bzw. ihnen entsprechende Pigmente mit sehr hohen spezifischen Oberflächen nach BET, insbesondere für LCD-Anwendungen hergestellt werden.

So können nach dem erfindungsgemäßen Verfahren spezifische Oberflächen nach BET der Metallverbindungen der Azoverbindungen der Formel (V) bzw. der Zusammensetzung mit mindestens einer eingelagerten Verbindung davon zwischen 180 und 240 m²/g, insbesondere zwischen 180 und 210 m²/g erhalten werden. Die spezifische Oberfläche wird nach DIN 66131 ermittelt: Bestimmung der spezifischen Oberfläche von Feststoffen durch Gasadsorption nach Brunauer, Emmett und Teller (B.E.T.)

Für bestimmte Anwendungen kann es sinnvoll sein, die nach dem erfindungsgemäßen Verfahren erhaltenen Metallverbindungen bzw. Einlagerungsverbindungen davon zu tempern, wie z.B. in der EP-A1-0994162 beschrieben. Durch das Tempern wird im Allgemeinen eine engere Partikelgrößenverteilung erhalten. Wie eingangs beschrieben, geht damit im allgemeinen aber auch eine Verringerung der spezifischen Oberfläche einher. Wird eine Temperung dennoch durchgeführt, besteht eine bevorzugte Ausführungsform darin, die hergestellte wässrige Suspension der Metallverbindung bzw. der Einlagerungsverbindung davon in wenigstens zwei pH-Stufen zu tempern. Dadurch kann gegenüber einer einstufigen Temperung eine deutlich verbesserte Farbstärke erhalten werden. Zweckmäßig erfolgt die mehrstufige Temperung in jeder Temperstufe bei Temperaturen zwischen 80 bis 125°C. Bevorzugt wird die mehrstufige Temperung in Gegenwart von Wasser und gegebenenfalls organischen Lösungsmitteln bei pH-Werten im Bereich von 0 bis 4 durchgeführt. Vorzugsweise liegt der pH-Wert von wenigstens einer Temperungsstufe zwischen 2 und 4, insbesondere zwischen 2,5 und 3,5. Vorzugsweise liegt der pH-Wert von einer zweiten Temperungsstufe zwischen 0 und 3, besonders bevorzugt zwischen 1 und 2,5. Vorzugsweise unterscheiden sich die pH-Werte von zwei Temperungsstufen um 0,5 bis 3 Einheiten, vorzugsweise um 1 bis 2 Einheiten. Vorzugsweise dauern wenigstens zwei Temperungsstufen unabhängig voneinander zwischen 0,25h und 24h, insbesondere zwischen 1h und 12h, ganz besonders bevorzugt zwischen 2h und 8h.

Die Verwendung der Impfkristalle sowie gegebenenfalls der Umpumpung kann aber auch bei Anwendung eines Temperverfahrens sinnvoll sein, da der Vorteil der geringeren Schwankung der Produktqualität bestehen bleibt und vor der Tempererung von einem höheren Niveau der spezifischen Oberfläche ausgegangen werden kann.

Das erfindungsgemäße Verfahren unter Verwendung der Impfkristalle führt nicht notwendigerweise zu spezifischen Oberflächen nach BET von mehr als 180 m²/g. Der Fachmann weiß nämlich, dass die Einstellung einer spezifischen Oberfläche auch von anderen Herstellparametem abhängt, wie z.B. der Herstellungstemperatur. Das erfindungsgemäße Verfahren führt jedoch unter sonst gleichen Herstellbedingungen mit höherer Reproduzierbarkeit zu einer höheren spezifischen Oberfläche nach BET der erfindungsgemäß hergestellten Metallverbindungen.

In einem besonders bevorzugten erfindungsgemäßen Verfahren befinden sich die Impfkristalle während oder vor Beginn der Azokupplung, besonders bevorzugt von Diazobarbitursäure mit Barbitursäure zur Azobarbitursäure, im Reaktionsmedium.

Besonders bevorzugte Edukte sind Barbitursäure und Diazobarbitursäure mit einem Gehalt an Impfkristallen, insbesondere an Salzen der Azobarbitursäure, vorzugsweise des Natrium-, Kalium- oder Nickel-Salzes, die auch bevorzugt als Interkalationsverbindungen, insbesondere des Melamins vorliegen können.

Ebenfalls bevorzugte Edukte sind Azobarbitursäure und ihre Salze, insbesondere die Natrium-, Dinatrium-, Kalium- und Dikalium-Salze mit einem Gehalt an Impfkristallen, insbesondere Nickel-Azobarbitursäure bevorzugt als Interkalationsverbindung insbesondere mit Melamin.

Das erfindungsgemäße Verfahren wird bevorzugt chargenweise bzw. als sogenanntes Batch-Verfahren durchgeführt. Der Begriff "Batch-Verfahren" meint, wie dem Fachmann gut bekannt ist, ein diskontinuierliches Verfahren. D.h., die Herstellung der Metall-Verbindungen wird nicht kontinuierlich sondern ansatz- bzw. chargenweise durchgeführt. Nach Durchführung eines Reaktionsansatzes wird das Produkt isoliert. Im Gegensatz dazu werden beim kontinuierlichen Verfahren kontinuierlich Edukte zugeführt und Produkt abgeführt.

Ein besonders bevorzugtes erfindungsgemäßes Verfahren besteht darin, dass Impfkristalle durch Zurücklassen der gewünschten Menge Produkt aus einer Vorläuferpartie in den Reaktor eingebracht oder dort belassen werden. Das Zurücklassen der gewünschten Menge Produkt kann insbesondere bei Serienproduktionen ökonomisch vorteilhaft eingesetzt werden.

Im Rahmen dieser Anmeldung werden die Metallverbindungen der Azoverbindungen der Formel (V), die vorzugsweise wenigstens eine Verbindung eingelagert enthalten und in Gegenwart von Impfkristallen hergestellt wurden, auch als erfindungsgemäß hergestellte Pigmente bezeichnet.

Einschlussverbindungen, Interkalationsverbindungen und feste Lösungen von Metallkomplexen an sich sind aus der Literatur bekannt. Sie werden ebenso wie deren Herstellung beispielsweise in der EP 0 074 515, der EP 0 073 463 , der EP 0994163 sowie der EP 0994162 (dort Seite 5, Zeile 40 bis Seite 7, Zeile 58) beschrieben. Insoweit kann vollinhaltlich auf die Aufzählung der in Frage kommenden Verbindungen in diesen Druckschriften Bezug genommen werden.

Die Menge an eingelagerter Substanz, die in das Kristallgitter des Metallkomplexes eingelagert werden kann, liegt in der Regel bei 5 % bis 200 Gew.-%, bezogen auf die Menge an Metallverbindung. Bevorzugt eingelagert werden 10 bis 100 Gew.-%. Es handelt sich hierbei um die Menge an eingelagerter Substanz, die durch geeignete Lösungsmittel nicht auswaschbar ist und die sich aus der Elementaranalyse ergibt. Naturgemäß kann auch mehr oder weniger als die genannte Menge an Substanz zugesetzt werden, wobei man gegebenenfalls darauf verzichten kann, einen Überschuß auszuwaschen. Bevorzugt sind Mengen von 10 bis 150 Gew.-% bezogen auf die Menge an Metallverbindung.

Die Herstellung der Metallverbindungen bzw. der Einlagerungsverbindungen davon erfolgt beispielsweise wie in EP 0 074 515, EP 0 073 463, EP 0994163 sowie der EP 0994162 beschrieben. Nach der Synthese der Azoverbindung wird im allgemeinen in Gegenwart der zu interkalierenden Verbindung mit einem Metallsalz komplexiert. Im Falle der technisch interessanten Interkalationsverbindungen des Azobarbitursäure-Nickel-Komplexes, erfolgen Komplexierung und Interkalation sowie auch die nachfolgende Isolierung zweckmäßig im sauren pH-Bereich.

Als Metallsalz kommen vorzugsweise wasserlösliche Metallsalze des Nickels in Frage, insbesondere Chloride, Bromide, Acetate, Nitrate usw.. Bevorzugt eingesetzte Metallsalze besitzen eine Wasserlöslichkeit von mehr als 20 g/l, insbesondere mehr als 50 g/l bei 20 °C.

Man kann auch Mischungen dieser Salze, die verschiedene der genannten Metalle enthalten können, verwenden. Die Verwendung von solchen Salzmischungen empfiehlt sich insbesondere für die Erzielung von Zwischentönen der farbigen Endprodukte.

In einer bevorzugten Ausführungsform wird das erfindungsgemäße Verfahren als Batch-verfahren in einem Reaktor, beispielsweise in einem Rührkesselreaktor, bevorzugt unter Anwendung einer Umpumpung durchgeführt. "Umpumpung" bedeutet dabei, dass Mittel vorgesehen sind, mit denen während der Herstellung dem Reaktor Inhalt entnommen und wiederzugeführt werden kann. Eine bevorzugte Ausgestaltung einer solchen Umpumpung besteht darin, dass der verwendete Reaktor, insbesondere ein Rührkessel ein vorzugsweise außerhalb des Reaktors liegendes Rohrleitungssystem aufweist. Das Rohrleitungssystem ist mit dem Reaktor bzw. Reaktorinhalt an mindestens zwei verschiedenen Stellen verbunden. Das Rohrleitungssystem weist Mittel auf, mit denen dem Reaktor an einer oder mehreren Stellen Reaktorinhalt entnommen werden kann und nach Passieren des Rohrleitungssystems an einer oder mehreren anderen Stellen wieder zugeführt werden kann. Derartige Mittel sind insbesondere Pumpen. Das erfindungsgemäß verwendete Umpumpungssystem weist bevorzugt Dosiereinrichtungen auf, die es ermöglichen, in das außerhalb des Reaktors liegende Rohrleitungssystem Reaktionspartner, beispielsweise Edukte, Lösungen von Edukten, Säuren, Basen etc. zu geben.

Ein besonders bevorzugtes erfindungsgemäßes Verfahren besteht darin, Säuren und Basen nicht direkt in den Reaktor sondern in das Umpumpungssystem zu dosieren. Ein besonders bevorzugtes erfindungsgemäßes Verfahren besteht darin, Reaktanden, Säuren und/oder Laugen bzw. Basen so zu dosieren, dass die Dosierzeit dem 0,2 fachen - 5 fachen eines theoretischen Gesamtumpumpcyclus, insbesondere dem 1 fachen - 2 fachen entspricht. Der theoretische Gesamtumpumpcyclus bedeutet den Zeitraum, innerhalb dessen das Volumen des Reaktorinhalts das Umpumpungssystem einmal passiert hat.

Es wird angenommen, dass die Umpumpung einen Bereich schafft, der eine vergleichsweise hohe Strömungsgeschwindigkeit aufweist. Diese Strömungsgeschwindigkeit liegt im allgemeinen höher als die Strömungsgeschwindigkeit im Rührkesselreaktor an Stellen geringer Rührwirkung, wie z.B. im Bereich oberhalb des obersten Rührblatts. Insbesondere bei Zudosierung im Bereich des Umpumpungssystems können durch die dort herrschende hohe Strömungsgeschwindigkeit lokale Konzentrationsspitzen vermieden werden. Weiterhin wird insgesamt eine bessere Durchmischung des Reaktorinhalts sichergestellt. Überraschend wird durch das erfindungsgemäße Verfahren ein Produkt erhalten, das eine noch höhere spezifische Oberfläche aufweist als ein Produkt, das ohne Umpumpungsverfahren hergestellt wurde. Weiterhin führt die Anwendung der Umpumpung zu einer zusätzlichen Verringerung der Schwankungen der Produktqualität. Es können mehrere Umpumpsysteme parallel eingesetzt werden.

Die bei der Herstellung erhaltene Suspension wird vorzugsweise filtriert und der so erhaltene Presskuchen kann gegebenenfalls nach Waschen mit Wasser getrocknet werden.

Dabei kommen einerseits übliche Trocknungsverfahren wie die Schaufeltrocknung usw. in Frage. Mit derartigen Trocknungsverfahren und anschließender Mahlung des Pigments auf übliche Weise werden pulverförmige Pigmente erhalten.

Bevorzugt wird der Presskuchen als wäßrige Slurry sprühgetrocknet. Der zu versprühende Slurry besitzt vorzugsweise einen Feststoffanteil von 10 bis 40 Gew.-%, insbesondere 15 bis 30 Gew.-%.

Die Erfindung betrifft weiterhin ein Verfahren zur Herstellung von Pigmentpräparationen, worin wenigstens eine erfindungsgemäß hergestellte Metallverbindung oder eine Einlagerungsverbindung davon und wenigstens ein Dispergiermittel vermischt werden. Diese Pigmentpräparationen dienen vorzugsweise der Einarbeitung in wässrige Systeme. Bezüglich geeigneter Dispergiermittel kann auf den eingangs erwähnten Stand der Technik verwiesen werden, insbesondere die EP-A1-0994164, Seite 8, Zeile 56 Seite 11, Zeile 23, deren Offenbarung zum Gegenstand dieser Anmeldung gehören. Besonders bevorzugt enthält die Pigment-Präparation mehr als 90 insbesondere mehr als 95, vorzugsweise mehr als 97 Gew.-% an Pigment (erfindungsgemäß hergestellte Metallverbindung + gegebenenfalls darin eingelagerte Verbindung(en)) und Dispergiermittel.

Die Erfindung betrifft weiterhin die erfindungsgemäß hergestellten Einlagerungsverbindungen mit einer spezifischen Oberfläche nach BET von mindestens 180 m²/g, wie zwischen 180 m²/g und 240 m²/g, insbesondere zwischen 180 und 210 m²/g sowie einen die genannten Einlagerungsverbindungen enthaltenden Photolack, der wenigstens ein photohärtbares Monomer und wenigstens einen Photoinitiator enthält. Die Erfindung betrifft weiterhin Farbfilter und daraus hergestellte Flüssigkristallanzeigen, die die erfindungsgemäß hergestellten Einlagerungsverbindungen mit einer spezifischen Oberfläche nach BET von mindestens 180 m²/g, wie zwischen 180 m²/g und 240 m²/g, enthalten. Bei der Herstellung der Farbfilter für Flüssigkristallanzeigen wird die erfindungsgemäß hergestellte Einlagerungsverbindung mit einer spezifischen Oberfläche nach BET von mindestens 180 m²/g, wie zwischen 180 m²/g und 240 m²/g, in einem organischen Lösungsmittel gegebenenfalls unter Zusatz eines Bindemittelharzes und/oder Dispergiermittels gemahlen, anschließend unter Zusatz von photohärtbaren Monomeren, Photoreaktionsstarter und ggf. weiterem Bindemittel und/oder Lösungsmittel zu einem Photolack verarbeitet wird, der im Anschluss daran mittels geeigneter Beschichtungsverfahren wie z.B. Roller-, Spray-, Spin-, Dip- oder Air-Knife-Coating auf ein geeignetes Substrat, im Allgemeinen eine Glasplatte aufgetragen wird, mittels Photomaske belichtet und anschließend gehärtet und zum fertigen farbigen Filter entwickelt wird.

Die Erfindung betrifft weiterhin bevorzugt die Verwendung der erfindungsgemäß hergestellten Einlagerungsverbindungen mit einer spezifischen Oberfläche nach BET von mindestens 180 m²/g, wie zwischen 180 m²/g und 240 m²/g, als Pigment für Farbfilter in Flüssigkristallanzeigen.

Die Erfindung betrifft weiterhin ein Verfahren zur Herstellung von Farbfiltern in Flüssigkristallanzeigen, das die Verwendung der erfindungsgemäß hergestellten Einlagerungsverbindungen mit einer spezifischen Oberfläche nach BET von mindestens 180 m²/g, wie zwischen 180 m²/g und 240 m²/g, umfasst.

Die erfindungsgemäß hergestellten Einlagerungsverbindungen davon oder Pigmentpräparationen eignen sich überdies hervorragend für alle Pigmentanwendungszwecke.

Zum Beispiel eignen sie sich zum Pigmentieren von Lacken aller Art für die Herstellung von Druckfarben, Leimfarben oder Binderfarben, für die Massefärbung von synthetischen, halbsynthetischen oder natürlichen makromolekularen Stoffen, wie z.B. Polyvinylchlorid, Polystyrol, Polyamid, Polyethylen oder Polypropylen. Sie können auch für die Spinnfärbung von natürlichen, regenerierten oder künstlichen Fasern, wie z.B. Cellulose-, Polyester-, Polycarbonat-, Polyacrylniril- oder Polyamidfasern, sowie zum Bedrucken von Textilien und Papier verwendet werden. Aus diesen Pigmenten können feinteilige, stabile, wässrige Pigmentierungen von Dispersions- und Anstrichfarben, die für die Papierfärbung, für den Pigmentdruck von Textilien, für den Laminatdruck oder für die Spinnfärbung von Viskose brauchbar sind, durch Mahlen oder Kneten in Gegenwart von nichtionogenen, anionischen oder kationischen Tensiden hergestellt werden. Die nach dem erfindungsgemäßen Verfahren hergestellten Pigmente eignen sich hervorragend für Ink Jet Anwendungen und aufgrund ihrer vergleichsweise hohen spezifischen Oberfläche nach BET für Farbfilter für Flüssigkristallanzeigen.

### Beispiele

### Erfindungs-Beispiel 1

190 g wasserfeuchte Paste von Diazobarbitursäure mit einem Trockengehalt von 81 %, entsprechend 154g trocken, werden in 3000 g Wasser mit einem Laborrührer verrührt. 600 mg Impfkristalle (Nickelsalz der Azobarbitursäure interkaliert mit Melamin mit einer spezifischen Oberfläche BET von 160 m²/g) werden zugegeben. Danach wird indirekt auf 80°C erwärmt und bei dieser Temperatur 134 g Barbitursäure eingetragen. Nach ca. 30 minütigem Nachrühren wird mit 30%iger Kaliumhydroxidlösung auf pH 5,0 gestellt. Anschließend wird 2 Stunden bei pH 5,0 und 80°C nachgerührt. Danach wird mit Wasser auf 5400 g verdünnt. Anschließend wird indirekt auf 90°C erwärmt und bei dieser Temperatur 252 g Melamin eingetragen. Danach werden 575 g 22,5%iger Nickelchloridlösung zugetropft. 90 Minuten wird nachgerührt, um eine möglichst vollständige Umsetzung zu erreichen. Dann wird mit 30%iger Kaliumhydroxidlösung auf pH 5,0 gestellt.

Anschließend wird die Pigment-Slurry (bis auf 6 g für Erfindungs-Beispiel 2) auf einer Saugnutsche isoliert, elektrolytfrei gewaschen, bei 80°C im Vakuumtrockenschrank getrocknet und in einer üblichen Labormühle ca. 2 Minuten gemahlen.

Der Versuch wird fünfmal wiederholt. Es werden Produkte mit einer BET-Oberfläche im Bereich von 180 bis 192 m²/g erhalten.

### Erfindungs-Beispiel 2

6 g der Pigment-Slurry einer Charge aus Erfindungs-Beispiel 1 als Impfkristallsuspension werden vorgelegt. 3000 g Wasser werden zugesetzt. 190 g wasserfeuchte Paste von Diazobarbitursäure mit einem Trockengehalt von 81 %, entsprechend 154 g trocken werden mit einem Laborrührer verrührt. Danach wird indirekt auf 80°C erwärmt und bei dieser Temperatur 134 g Barbitursäure eingetragen. Nach ca. 30 minütigem Nachrühren wird mit 30%iger Kaliumhydroxidlösung auf pH 5,0 gestellt. Anschließend wird 2 Stunden bei pH 5,0 und 80°C nachgerührt. Danach wird mit Wasser auf 5400 g verdünnt. Anschließend wird indirekt auf 90°C erwärmt und bei dieser Temperatur 252 g Melamin eingetragen. Danach werden 575 g 22,5%iger Nickelchloridlösung zugetropft. 90 Minuten wird nachgerührt, um eine möglichst vollständige Umsetzung zu erreichen. Dann wird mit 30%iger Kaliumhydroxidlösung auf pH 5,0 gestellt.

Anschließend wird auf einer Saugnutsche isoliert, elektrolytfrei gewaschen, bei 80°C im Vakuumtrockenschrank getrocknet und in einer üblichen Labormühle ca. 2 Minuten gemahlen.

Man erhält ein Produkt mit einer BET-Oberfläche von 185 m²/g.

### Vergleichs-Beispiel 1

190 g wasserfeuchte Paste von Diazobarbitursäure mit einem Trockengehalt von 81 %, entsprechend 154g trocken, werden in 3000 g Wasser mit einem Laborrührer verrührt. Danach wird auf 80°C erwärmt und bei dieser Temperatur 134 g Barbitursäure eingetragen. Nach ca. 30 minütigem Nachrühren wird mit 30%iger Kaliumhydroxidlösung auf pH 5,0 gestellt. Anschließend wird 2 Stunden bei pH 5,0 und 80°C nachgerührt. Danach wird mit Wasser auf 5400 g verdünnt. Anschließend wird indirekt auf 90°C erwärmt und bei dieser Temperatur 252 g Melamin eingetragen. Danach werden 575 g 22,5%iger Nickelchloridlösung zugetropft. 90 Minuten wird nachgerührt, um eine möglichst vollständige Umsetzung zu erreichen. Dann wird mit 30%iger Kaliumhydroxidlösung auf pH 5,0 gestellt.

Anschließend wird die Pigment-Slurry auf einer Saugnutsche isoliert, elektrolytfrei gewaschen, bei 80°C im Vakuumtrockenschrank getrocknet und in einer üblichen Labormühle ca. 2 Minuten gemahlen.

Der Versuch wird fünfmal wiederholt. Es werden Produkte mit einer BET-Oberfläche im Bereich von 143 bis 177 m²/g erhalten.

### Vergleichs-Beispiel 2

In einem 20m³ Reaktor mit Mantelheiz-/kühl-System, Rührer, Stromstörer und Umpumpsystem werden 6.000 Liter 80°C heißes Wasser bei einer Rührgeschwindigkeit von 20 U/min vorgelegt. 380 kg wasserfeuchte Paste von Diazobarbitursäure mit einem Trockengehalt von 81 %, entsprechend 308 kg trocken, werden eingetragen.

Die Temperatur wird auf 80°C gehalten und bei dieser Temperatur 268 kg Barbitursäure eingetragen. Es wird mit einer Umpumpung gearbeitet, die auf 15m³/h eingestellt ist. Nach 1 Stunde Umpumpung wird mit 30%iger Kaliumhydroxidlösung innerhalb 30min auf pH 5,0 gestellt, wobei die Kaliumhydroxidlösung in die Umpumpung dosiert wird. Anschließend wird 2 Stunden bei pH 5,0 und 80°C unter Umpumpen nachgerührt. Danach wird mit Wasser auf 15.000 Liter verdünnt. Anschließend wird auf 90°C erwärmt und bei dieser Temperatur 500 kg Melamin eingetragen. Die Umpumpung wird auf 30m³/h gestellt. Danach werden 1.150 kg 22,5%iger Nickelchloridlösung über den Umpumpkreislauf innerhalb 30min eindosiert. 90 Minuten wird unter Umpumpen nachgerührt, um eine möglichst vollständige Umsetzung zu erreichen. Dann wird mit 30%iger Kaliumhydroxidlösung innerhalb 30min auf pH 5,0 gestellt, wobei die Kaliumhydroxidlösung in die Umpumpung eindosiert wird.

Danach wird über eine Dosierung von Salzsäure in den Umpumpkreislauf innerhalb 30 min auf pH 2,5 gestellt. Die Temperatur wird auf 98°C erhöht und 4 Stunden getempert. Danach wird mit 30%iger Kaliumhydroxidlösung innerhalb 30min auf pH 5,0 gestellt, wobei die Kaliumhydroxidlösung in den Umpumpkreislauf eindosiert und die Temperatur auf 80°C geregelt wird.

Der anbackungsfreie Reaktor läßt sich sehr leicht praktisch vollständig entleeren. Die homogene Pigment-Slurry wird auf einer Filterpresse isoliert, elektrolytfrei gewaschen und bei 80°C getrocknet. Man erhält ein sehr einheitliches Produkt mit sehr enger Partikelgrößenverteilung und einer BET-Oberfläche von 167 m²/g.

### Erfindungs-Beispiel 3

Man lässt in dem Ansatz von Vergleichsbeispiel 2 etwa 20 Liter der Produktsuspension als Impfkristalle im Reaktor zurück und arbeitet dann erneut wie in Vergleichsbeispiel 2 beschrieben. Man erhält ein sehr einheitliches Produkt mit sehr enger Partikelgrößenverteilung und einer BET-Oberfläche von 182 m²/g.

## Patentansprüche

1. Verfahren zur Herstellung einer Nickel-Verbindung einer Azo-Verbindung die in Form ihrer freien Säure der Formel (V) oder einer tautomeren Form davon entspricht 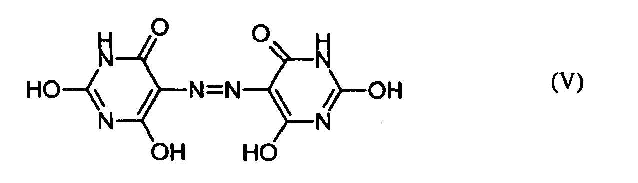 und die Melamin eingelagert enthält, **dadurch gekennzeichnet, dass** die Herstellung in Gegenwart von Impfkristallen erfolgt.

2. Verfahren zur Herstellung der Einlagerungsverbindung gemäß Anspruch 1, **dadurch gekennzeichnet, dass**
die eingesetzten Impfkristalle die gleiche chemische Struktur aufweisen wie die herzustellende Einlagerungsverbindungen.

3. Verfahren zur Herstellung der Einlagerungsverbindung gemäß einem der Ansprüche 1 bis 2, **dadurch gekennzeichnet, dass** die Herstellung in Gegenwart von 1 ppm - 10.000 ppm, vorzugsweise von 10 ppm - 5.000 ppm, ganz besonders bevorzugt von 50 ppm - 3.000 ppm, insbesondere von 100 ppm - 2.000 ppm an Impfkristallen, jeweils bezogen auf die theoretisch erhältliche Menge der herzustellenden Metallverbindung, erfolgt.

4. Verfahren zur Herstellung der Einlagerungsverbindung gemäß einem der Ansprüche 1 bis 3, **dadurch gekennzeichnet, dass** sich die Impfkristalle während oder vor der Azokupplung im Reaktionsmedium befinden.

5. Verfahren zur Herstellung der Einlagerungsverbindung gemäß einem der Ansprüche 1 bis 4, **dadurch gekennzeichnet, dass** eine spezifische Oberfläche nach BET der Metallverbindungen oder der Einlagerungsverbindung davon von mindestens 160 m²/g erhalten wird.

6. Verfahren zur Herstellung der Einlagerungsverbindung gemäß einem der Ansprüche 1 bis 5, **dadurch gekennzeichnet, dass** die nach dem Verfahren gemäß einem der Ansprüche 1 bis 5 hergestellte Einlagerungsverbindung als wässrige Slurry sprühgetrocknet wird.

## Claims

1. Process for preparing a nickel compound of an azo compound which conforms in the form of its free acid to the formula (V) or to a form tautomeric therewith 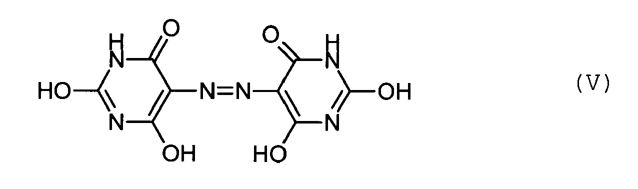 and which contains melamine as guest compound, **characterized in that** the preparation takes place in the presence of seed crystals.

2. Process for preparing the host-guest compound according to Claim 1, **characterized in that** the seed crystals used have the same chemical structure as the host-guest compounds to be prepared.

3. Process for preparing the host-guest compound according to either of Claims 1 and 2, **characterized in that** the preparation takes place in the presence of 1 ppm - 10 000 ppm, preferably of 10 ppm - 5000 ppm, very preferably of 50 ppm - 3000 ppm, in particular of 100 ppm - 2000 ppm of seed crystals, based in each case on the theoretically obtainable amount of the metal compound to be prepared

4. Process for preparing the host-guest compound according to one of Claims 1 to 3, **characterized in that** the seed crystals are in the reaction medium during or prior to azo coupling.

5. Process for preparing the host-guest compound according to one of Claims 1 to 4, **characterized in that** a BET specific surface area of the metal compounds or of the host-guest compound thereof is obtained of at least 160 m²/g.

6. Process for preparing the host-guest compound according to one of Claims 1 to 5, **characterized in that** the host-guest compound that is prepared by the process according to one of Claims 1 to 5 is spray-dried in the form of an aqueous slurry.

## Revendications

1. Procédé pour la préparation d'un composé du nickel d'un composé azo qui correspond, sous forme de son acide libre, à la formule (V) ou à une forme tautomère de celle-ci 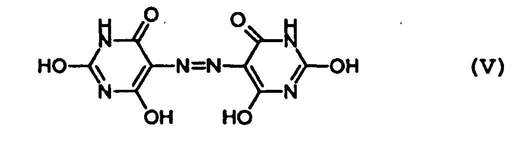 et qui contient de la mélamine sous forme incorporée, **caractérisé en ce que** la préparation a lieu en présence de cristaux d'ensemencement.

2. Procédé pour la préparation du composé d'incorporation selon la revendication 1, **caractérisé en ce que** les cristaux d'ensemencement utilisés présentent la même structure chimique que les composés d'incorporation à préparer.

3. Procédé pour la préparation du composé d'incorporation selon l'une quelconque des revendications 1 à 2, **caractérisé en ce que** la préparation a lieu en présence de 1 ppm-10 000 ppm, de préférence de 10 ppm-5000 ppm, de manière tout particulièrement préférée de 50 ppm-3000 ppm, en particulier de 100 ppm-2000 ppm de cristaux d'ensemencement, à chaque fois par rapport à la quantité pouvant être obtenue théoriquement du composé métallique à préparer.

4. Procédé pour la préparation du composé d'incorporation selon l'une quelconque des revendications 1 à 3, **caractérisé en ce que** les cristaux d'ensemencement se trouvent dans le milieu réactionnel pendant ou avant le couplage azo.

5. Procédé pour la préparation du composé d'incorporation selon l'une quelconque des revendications 1 à 4, **caractérisé en ce qu'**on obtient une surface spécifique selon BET des composés métalliques ou du composé d'incorporation de celui-ci d'au moins 160 m²/g.

6. Procédé pour la préparation du composé d'incorporation selon l'une quelconque des revendications 1 à 5, **caractérisé en ce que** le composé d'incorporation préparé sous forme de suspension aqueuse selon le procédé selon l'une quelconque des revendications 1 à 5 est séché par pulvérisation.
